# EUROPÄISCHE PATENTSCHRIFT

(11) **EP 1 360 492 B1**
(45) Veröffentlichungstag und Bekanntmachung des Hinweises auf die Patenterteilung: **03.12.2008**
(21) Anmeldenummer: 02716748.5
(22) Anmeldetag: 08.02.2002
(51) Int. Cl.: G01N 33/543

(54) **PROBENTRÄGER FÜR CHEMISCHE UND BIOLOGISCHE PROBEN**
SAMPLE SUPPORT FOR CHEMICAL AND BIOLOGICAL SAMPLES
PORTE-ECHANTILLON POUR ECHANTILLONS CHIMIQUES ET BIOLOGIQUES

(30) Priorität: 08.02.2001 DE 10105711
(43) Veröffentlichungstag der Anmeldung: 12.11.2003
(73) Patentinhaber: ibidi GmbH, 82152 Martinsried (DE)
(72) Erfinder: RÄDLER, Ulf, 80805 München (DE)
(74) Vertreter: Grünecker, Kinkeldey, Stockmair & Schwanhäusser Anwaltssozietät
(86) Internationale Anmeldenummer: PCT/EP2002/001343
(87) Internationale Veröffentlichungsnummer: WO 2002/063304

(56) Entgegenhaltungen:
- WO-A-00/33079
- WO-A-00/40750
- WO-A-00/78821
- WO-A-01/54810
- WO-A-01/94032
- DE-A- 4 026 992
- US-A- 5 919 523

## Beschreibung

Die Erfindung betrifft einen Probenträger zur Untersuchung chemischer und biologischer Proben und dessen Verwendung zur Immobilisierung von Proben.

Die Untersuchung von chemischen und biologischen Materialien wie Therapeutika, Umweltproben, DNA, RNA, Proteinen, Antigenen, Antikörpern oder auch Zellen und deren Fragmenten spielt eine wesentliche Rolle in der heutigen Forschung. Ihre Eigenschaften werden in speziellen Erkennungsuntersuchungen, den sogenannte Wechselwirkungsanalysen, wie zum Beispiel DNA-Hybridisierungstests oder auch Tests der Antikörper-Antigenwechselwirkungen, im Medikamentenscreening und in der Umweltanalytik, untersucht.

Die Probenträger, mit denen diese Untersuchungen bisher durchgeführt werden, bestehen meist aus Glas. Glas erfüllt hohe optische Anforderungen und ist gegen den Angriff aggressiver Chemikalien wie Säuren, Basen, organischen Lösungsmitteln usw. nahezu inert. Deshalb haben sich für die oben angesprochenen Untersuchungen in der Forschung und Industrie Glasträger als Probenplattform etabliert.

Glas ist in der Verarbeitung aufwendig und zudem ein empfindliches Material. Die Formgebung von Glas ist ein technologisch umfangreicher Prozess, der im allgemeinen durch Strukturätzung oder Pressung erfolgt. Dabei können die optisch hochwertigen Eigenschaften des Glases, die für viele Untersuchungsmethoden erforderlich sind, verloren gehen. Zunehmend werden daher für Probenträger Kunststoffe verwendet, da diese nicht nur billiger und robuster sind als Glas, sondern auch leichter verarbeitet werden können. Die Formgebung bei Kunststoffen kann zum Beispiel durch Spritzguss oder Heißprägung erreicht werden. In den letzten Jahren sind neuartige Kunststoffe entwickelt worden, deren optische Eigenschaften für die oben angesprochenen Analysesysteme ausreichend sind. Zu derartigen Kunststoffen zählen sowohl cyclische Olefinpolymere, deren Kopolymerisate und Derivate der Norbonenfamilie. Solche Polymere besitzen hervorragende optische Eigenschaften und werden zum Beispiel auch im Bereich der Linsenherstellung verwendet.

Für die Untersuchung mancher Probeneigenschaften ist ein Flusssystem erforderlich, um die Proben z. B. mit frischem Kulturmedium zu versorgen oder sie mit Kohlendioxid zur Stabilisierung des pH-Wertes begasen zu können. Hierfür wurden Probenkammern mit einem Kanalsystem entwickelt, in dem die Proben luftdicht und steril verschlossen und über Zuführungen mit einem gewünschten Medium versorgt werden können.

Aus der US 5,665,599 ist ein Kammersystem mit einzelnen abgeschlossenen Abteilen bekannt, die durch Zuführungen von außen mit Flüssigkeiten oder Gasen versorgt werden können. Ferner wurde in der WO 98/45693 eine Plastikkammer mit Mikrokanälen offenbart, wobei die Größe der Kanäle im kapillaren Bereich liegt.

Für einige Versuche an Proben mit den eingangs genannten Untersuchungsmethoden ist es wünschenswert, die Proben zu immobilisieren, d. h. die einzelnen Moleküle einer Probe auf der Oberfläche des Probenträgers zu fixieren. Hierfür werden meist funktionalisierte Oberflächen mit freien Bindungsstellen verwendet; an die die Probe gekoppelt werden kann.

Glas lässt sich unter geeigneten Bedingungen oberflächenfunktionalisieren, wobei bestimmte chemische Verbindungen an die Oberfläche gebunden werden. Das bedeutet, dass die Oberfläche des Glases in einem aufwendigen Bearbeitungsabschnitt verändert wird. Dies wird durch aggressive Methoden wie z.B. durch Organsilansierung durchgeführt. Anschließend ist es möglich, eine direkte Anbindung der Untersuchungsproben an die Oberfläche zu erzielen oder eine Schicht mit freien Bindungen auf das Glas aufzubringen. Diese funktionelle Schicht kann z. B. eine Metallschicht sein und die fortgesetzte Funktionalisierung der Oberfläche ermöglichen.

Es ist beispielsweise ein Probenträger der Firma Biacore bekannt, bei dem ein Glasträger mit Gold beschichtet wird. Die Funktionalisierung ist in diesem Fall durch Kopplung von Organothiolen auf der Goldschicht gegeben. An diese kann die zu untersuchenden Probe anbinden und wird dadurch immobilisiert.

Insgesamt ist die Funktionalisierung eines Probenträgers auf diese Weise ein zeitaufwendiger und komplizierter Prozess, da verschiedenste Arbeitsschritte mit einer hohen Präzession auszuführen sind. Zudem sind bisher nur begrenzte Möglichkeiten vorhanden verschiedenartige Funktionalisierungen zu erzielen, um z. B. die Funktionalisierung für eine bestimmte Probenart zu optimieren oder das Verhalten der Probe bei unterschiedlichen Wechselwirkungspartnern zu untersuchen. Außerdem ist es mit herkömmlichen Funktionalisierungsmethoden nicht möglich, eine genau definierte Funktionalisierung mit beispielsweise einer bestimmten Konzentration an freien Bindungen auf dem Probenträger zu ermöglichen. Letztlich ist bisher auch kein Probenträger mit einem Kanalsystem bekannt, dessen Kanäle eine Funktionalisierung aufweisen.

Aus der DE 4026992 ist ein Verfahren zur Herstellung von Trägersystemen für biologischen aktive Materialien bekannt, wobei eine Polymer-Schicht mit der Oberfläche mit zur kovalenten Bindung geeigneten funktionalen Gruppen bereit gestellt wird.

Aus der US 5,919,823 sind Substrate zur Oligomersynthese bekannt.

Die WO00/78821 zeigt Methoden und Reagenzien für multivalente Arrays.

Aus der WO00/33079 ist ein Verfahren zum Herstellen von Nanopartikeln mit wenigstens einer Polymerhülle bekannt.

Ein Verfahren zum Sequenzieren von DNA mit Hilfe eines mikrofluidischen Geräts ist aus der WO00/40750 bekannt.

Die nachveröffentlichte WO01/94032 offenbart Verfahren zur Herstellung von als Ausgangsprodukte für Mikroarrays die einen oberflächenfunktionalisierten Trägem zur Mobilisierung von Biomolekülen.

Es ist daher die Aufgabe der vorliegenden Erfindung einen Probenträger mit einer Funktionalisierung zu schaffen, die an verschiedene Untersuchungsanforderungen angepasst ist und geeignete optische Eigenschaften für unterschiedliche optische Untersuchungsmethoden aufweist, und dessen Verwendung die Immobilisierung chemischer und biologischer Proben ermöglicht, die Analyse der Probe beschleunigt und deren Präparation erleichtert.

Diese Aufgabe ist durch die Merkmale des Patentanspruchs 1 gelöst. Demnach ist ein Probenträger zur Untersuchung chemischer und biologischer Proben vorgesehen, der eine Oberfläche aus Kunststoff oder einem Kunststoffverbund und freie Bindungsstellen an wenigstens einem Bereich der Oberfläche aufweist, die durch eine Funktionalisierung gebildet werden, die aus wenigstens einer funktionellen Gruppe besteht, und durch dessen Verwendung die Proben immobilisiert werden.

Vorteilhafte Ausgestaltungen der Erfindung gehen aus den Unteransprüchen hervor.

Ein Probenträger nach der vorliegenden Erfindung ermöglicht eine einfache Analyse verschiedener Wechselwirkungen von chemischen und biologischen Probenmaterialien, insbesondere von Makromolekülen wie Proteinen, Antigenen-Antikörpern, und z.B. Zellfragmenten, sowie Langzeitstudien an diesen Proben unter optimalen mikroskopischen und fluoreszenztechnischen Bedingungen.

Die funktionellen Gruppen des Probenträgers werden schon bei der Polymerisation des Kunststoffes auf der Oberfläche ausgebildet. Dabei sind diese Gruppen im allgemeinen chemisch an die Kunststoffpolymere gebunden. Es sind daher keine weiteren Schritte zur Erzeugung einer Funktionalisierung der Oberfläche erforderlich.

Bevorzugt wird die Funktionalisierung des Probenträgers durch eine Carboxylsäuregruppe (-COOH), eine Aminogruppe (-NH₂), eine Thiolgruppe (-SH), eine Hydroxygruppe (-OH), eine Aldehydgruppe (-CH₂O), eine Säurehalogenidgruppe (-COX) oder eine Polyethylenglycolgruppe gebildet. Die ursprüngliche Funktionalisierung kann aber auch durch zusätzliche chemische Modifikationen erweitert werden, indem weitere reaktive Gruppen an die funktionellen Gruppen angebunden werden.

Hierfür können z.B. Imminodiessigsäure- oder Nitrilotriessigsäurederivate oder Biotinderivate verwendet werden, aber auch andere üblich Materialien zur funktionalen Immobilisierung, wie z. B. Metallschichten.

Da der Probenträger für die erwähnten optischen Untersuchungsmethoden geeignet sein soll, wird ein Kunststoff verwendet, der transparent und für weite Bereiche des ultravioletten Lichtes durchlässig ist sowie keine doppelbrechenden oder Autofluoreszenzeigenschaften aufweist. Vorzugsweise wird der Kunststoff aus der Gruppe der cyclischen Olefinpolymere, der Norbonenpolymere, der cyclischen Olefincopolymere und/oder deren verwandten Polymergruppen ausgewählt ist und/oder Polypropylen ist. Durch eine vorbestimmte Zusammensetzung des verwendeten Kunststoffes kann die Zahl der freien Bindungsstellen variiert und den Versuchsanforderungen angepasst werden. Dadurch ist es möglich auf unkomplizierte Weise für die Oberfläche des Probenträger freie Bindungsstellen auszuwählen, die auf die zu untersuchende Probe abgestimmt sind und mit dieser eine gewünschte Bindung eingehen.

Der Probenträger ist als Kammer mit wenigstens einem Kanal ausgebildet, wobei zumindest die Oberfläche des Kanalbodens aus einem Kunststoff oder einem Kunststoffverbund mit einer Funktionalisierung durch funktionelle Gruppen besteht. Natürlich ist es auch möglich, die gesamte innere Oberfläche des Kanals mit einer Funktionalisierung zu versehen. Dabei kann die Kammer mehrere Kanäle mit jeweils einer unterschiedlichen Funktionalisierung aufweisen, um die Immobilisierung verschiedener Wechselwirkungspartner in verschiedenen Kanälen der gleichen Kammer zu ermöglichen. Zudem ist es u. a. vorteilhaft in einem einzelnen Kanal mehrere Bereiche mit unterschiedlicher Funktionalisierung zur Verfügung zustellen. Dadurch können verschiedene Arten der Verankerung des immobilisierten Partners ermöglicht werden, zum Beispiel für eine säureseitige, aminoseitige oder thiolseitige Fixierung eines Proteins. Durch derartige vielseitige Oberflächen mit unterschiedlichen Funktionalisierungen werden multiple Wechselwirkungsanalysen möglich.

Ferner ist die Kammer luftdicht und steril verschließbar und die Kanäle der Kammer weisen Zugriffsöffnungen auf. An einen Kanal können durch diese Zugriffsöffnungen Zu- und Abläufe von Flüssigkeiten oder Gasen realisiert werden. Dadurch können Substanzen zugeführt werden, die entweder der eigentlichen Immobilisierung eines Probenmaterials, der Versorgung des Probenmaterials oder der Durchführung der Untersuchung dienen. Derartige Substanzen sind beispielsweise Kohlendioxid, diverse Puffer, verschiedene Reaktanten, etc. Vor allem um Langzeitmessungen an den oben genannten Systemen ermöglichen zu können, müssen die biologischen Proben nach ihrer Immobilisierung mit versorgenden Medien versetzt werden.

Bei der Verwendung eines erfindungsgemäßen Probenträgers, wird eine Probe auf eine funktionalisierte Oberfläche des Probenträgers gebracht und bindet an die freien Bindungsstellen der funktionellen Gruppe, wodurch die Probe immobilisiert wird. Eine derartige Bindung kann kovalenter, ionischer, koordinativer oder komplexbildender Natur sein. Für die meisten Untersuchungsmethoden wird eine kovalente Bindung bevorzugt. Natürlich ist es auch möglich statt der Probe, den jeweiligen Wechselwirkungspartner der Probe zu immobilisieren, wodurch das Verhalten einer auf dem Wechselwirkungspartner beweglichen Probe analysiert werden kann.

Durch die Verwendung einer Probenträgerkammer mit Kanälen können in den einzelnen Kanälen unterschiedliche Untersuchungsvoraussetzungen gleichzeitig verwirklicht werden, z. B. können gleichzeitig mehrere verschiedene Proben oder eine bestimmte durch verschiedene Funktionalisierungen immobilisierte Proben analysiert werden.

Durch die Immobilisierung der Probe werden Untersuchungen ermöglicht, die durch Fluoreszenztechniken, mikroskopische Techniken oder fluoreszenzmikroskopische Techniken ausgelesen werden können. Ferner werden Langzeitstudien an den immobilisierten Materialien ermöglicht. Solche Langzeitstudien sind besonders im Bereich der Pharmaforschung, der Gewebeuntersuchung und im Bereich der Medikamentenuntersuchung von größter Wichtigkeit.

Die Erfindung ist in Ausführungsbeispielen anhand der Zeichnungen näher erläutert. In diesen stellen dar:
Fig. 1: eine schematische Darstellung eines Probenträgers nach der vorliegenden Erfindung und
Fig. 2: eine schematische Darstellung einer Probenträgerkammer nach einer bevorzugten Ausführungsform der Erfindung.

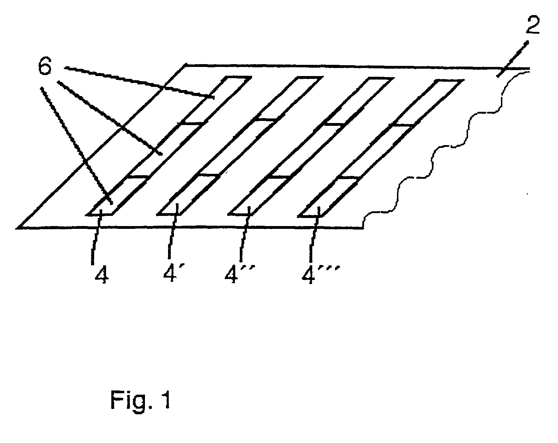

In.Figur 1 ist die Oberfläche 2 eines Probenträgers aus Kunststoff nach der vorliegenden Erfindung gezeigt. Die Oberfläche 2 weist mehrere längliche Bereiche 4 mit unterschiedlicher Funktionalisierung durch funktionelle Gruppen auf. Die Bereiche 4 können aber auch eine beliebige andere Form aufweisen. Die einzelnen Bereiche sind in aufeinanderfolgende Abschnitte 6 mit jeweils unterschiedlicher Funktionalisierung unterteilt. Hierfür kann eine Kombination verschiedener Kunststoffe verwendet werden. Es ist auch möglich einen oder mehrere der Abschnitte mit einer Metallfunktionalisierung, beispielsweise aus Gold, zu versehen.

Diese Funktionalisierung wird z. B. dadurch erzielt, dass von vornherein ein funktionaler Kunststoff verwendet wird, wodurch die Oberfläche des Kunststoffs von Anfang an eine Funktionalisierung aufweist. Es kann auch ein Kunststoffverbund verwendet werden. Natürlich kann die Funktionalisierung auch nachträglich erfolgen, wobei der Kunststoff mit der Funktionalsierung in gelöster Form auf eine ursprüngliche Oberfläche aufgebracht wird und sich dort abscheidet. Derart ist es auch möglich, weitere reaktive Gruppen an die freien Bindungen der funktionelle Gruppe zu binden, wodurch die Eigenschaften der Funktionalisierung der Oberfläche noch weiter veränderbar sind.

Der Probenträger kann daher insgesamt aus einem einheitlichen Kunststoff gemäß der Erfindung bestehen oder aus einem beliebigen Trägermaterial mit nachträglich aufgebrachter erfindungsgemäßer Kunststoffoberfläche mit einer Funktionalisierung gebildet werden.

Um die Anforderungen optischer Untersuchungsmethoden zu erfüllen, ist es wesentlich, dass der Probenträger eine geringe Dicke aufweist. Der Probenträger der vorliegenden Erfindung weist eine einheitliche Dicke von 40 - 400 µm auf; vorzugsweise 170 µm, da herkömmliche Objektive auf dieses Maß normiert sind.

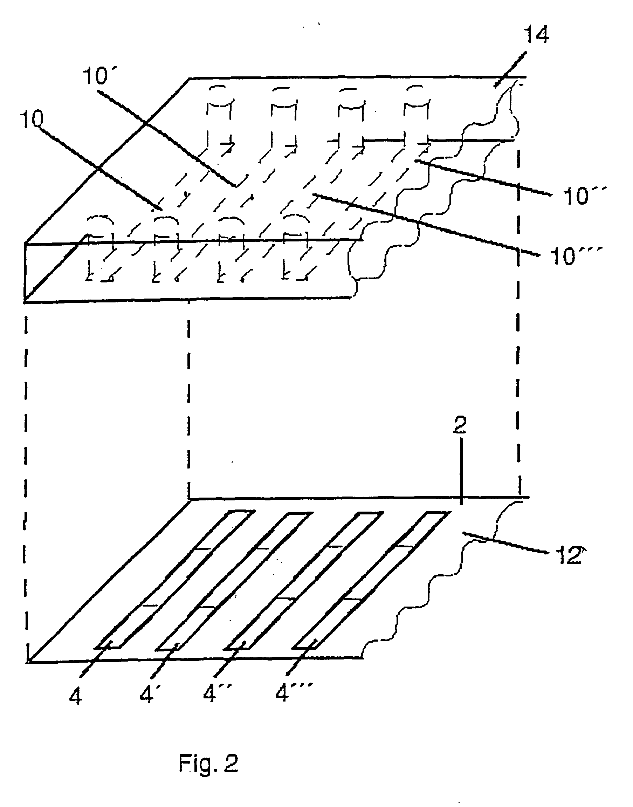

In Figur 2 ist ein als Kammer 8 ausgebildeter Probenträger aus Kunststoff mit mehreren Kanälen 10, 10', 10'' und 10'" gezeigt, der aus einem unteren Teil 12 und einer Abdeckung 14 besteht. Mit einer derartigen Kammer zum Immobilisieren und Analysieren von Proben und deren Wechselwirkungspartnern können mehrere unterschiedliche Proben gleichzeitig in der Kammer präpariert werden, wodurch z. B. die Durchführung von Messreihen erleichtert wird und weniger Zeit benötigt. Die Kammer weist als Boden einen unteren Teil 12 mit einer Oberfläche 2 auf, wie sie in Figur 1 beschrieben wurde. Die Kanäle werden durch Aufsetzten einer Abdeckung 14 auf das untere Teil 12 gebildet. Hierfür sind in der Abdeckung 14 Wände 16 vorgesehen, die mit dem unteren Teil 12 abschließen, sowie Zugriffsöffnungen 18, die bevorzugt oben auf der Abdeckung angeordnet und z. B. durch Teflondeckel verschießbar sind. Nach dem Aufsetzen der Abdeckung 14 auf den unteren Teil 12 bilden die funktionalisierten länglichen Bereiche 4 der Oberfläche 2 den Boden der Kammern 10, 10', 10" und 10'''. Diese Bereiche sind die für die mikroskopischen, fluoreszenztechnischen, fluoreszenzmikroskopischen Untersuchungen hauptsächlich relevanten Flächen.

Zur Herstellung der Probenträgerkammer werden der untere Teil 12 und die Abdeckungen 14 miteinander verbunden, wofür gängige kunststoffverarbeitende Verfahren wie Lösungsmittelverkleben und Heißverkleben verwendet werden. Die Länge eines dadurch entstehenden Kanals beträgt 1- 20 cm, bevorzugt 1-12 cm. Die Kanalbreite und die Kanalhöhe betragen 10 µm -20 mm, bevorzugt 10 - 500 µm. Die Höhe der Kammer beträgt 0,4-25 mm, bevorzugt 1 - 5 mm und ist über die gesamte Kammer einheitlich. Durch diese geringe Bauhöhe ist es möglich, dass die Beleuchtung der Probe nicht durch störendes Streulicht behindert wird. Der untere Teil 12 wird durch eine dünne Folie aus einem erfindungsgemäß funktionalisierten hochtransparenten Kunststoff gebildet, wodurch sich vor allem für inverse Mikroskopietechniken Vorteile ergeben.

Auch die Wände 16 der Kanäle 10 und deren Decke, d. h. die Unterseite der Abdeckung, können eine Funktionalisierung besitzen und eine Immobilisierung der Proben erlauben. Diese Funktionalisierung kann gleicher Art wie auf dem unteren Teil oder anderer Natur sein.

Um die Probenträgerkammer als Flusssystem zu nutzen, werden die Zugriffsöffnungen als Zulauf 18 bzw. Ablauf 18' für Flüssigkeiten oder Gasen verwendet. Es ist auch möglich in der Kammer eine gemeinsame Zugriffsöffnung als Zulauf und entsprechend eine gemeinsame Zugriffsöffnung als Ablauf für mehrere Kanäle vorzusehen, indem die Kanäle in einer gemeinsamen Öffnung zusammenlaufen.

Durch das Vorhandensein mehrerer Kanäle in dem Probenträger, wird die Ausbildung bevorzugt paralleler oder senkrechter zueinander verlaufender Strukturen ermöglicht, z.B. durch einen Kreuzkanal. Es können in einem Kanal mehrere verschiedene Immobilisierungsstrecken nebeneinander auf dem Kunststoff realsisiert werden, bevorzugt 1-1000, weiter bevorzugt 1-100, was zum Beispiel in Multifunktionsassays, bei Blutproben, bei Krankheitsanalysen von Bedeutung ist.

Gemäß einer Verwendung der Probenträgerkammer werden zu untersuchende biologische oder chemische Proben durch Bindung an die freien Bindungsstellen der Oberfläche immobilisiert. Bevorzugt wird hierfür eine Kammer mit mehreren Kanälen verwendet, die jeweils eine Funktionalisierung mit einer unterschiedlichen funktionellen Gruppe aufweisen.

So ist z. B. in Figur 2 der Kanal 10 mit einer Säurefunktionalisierung versehen, die eine Immobilisierung z. B. eines Proteins A über freie Aminogruppen ermöglicht. Der Kanal 10' ist mit einer Aminofunktionalisierung versehen, durch die das gleiche Protein A oder ein anderes Protein B über eine Säuregruppe immobilisiert wird. Durch eine NTA Funktionalisierung der Kanals 10" können die gleichen oder ein weiteres Protein C über einen His-Tag immobilisiert werden, usw.. Die immobilisierten Proben A,B,C,... können auf ihre biologische Aktivität nach der Immobilisierung getestet werden und sind nun z. B. für eine Wechselwirkungsuntersuchung präpariert.

Bei der Untersuchung zur Wechselwirkung von A,B,C,... mit einem oder mehreren freien Wechselwirkungspartnern A1,B1,C1,... , werden diese über die Zugriffsöffnungen in den jeweiligen Kanal eingespült. Die Wechselwirkungsanalyse kann in einem dynamischen oder statischen Zustand erfolgen. Sind die immobilisierten Proben nicht kovalent auf der Oberfläche fixiert, können sie nach erfolgter Messung wieder vollständig von der Oberfläche entfernt werden. Anschließend kann die Oberfläche zur erneuten Immobilisierung einer Probe verwendet werden. Dieser Prozess ist mehrfach wiederholbar, bevorzugt 1 - 10 mal.

Bei einer weiteren Verwendung einer Probenträgerkammer mit verschiedenen funktionellen Gruppen in den Abschnitten 6 eines einzelnen Kanals 10 werden parallele Untersuchungen immobilisierter Partner A,H,C... ermöglicht. Dadurch wird die Zeit für die Durchführung einer Messung weiter reduziert und die Messung ist weniger fehleranfällig, da weniger Arbeitsgänge erforderlich sind.

## Patentansprüche

1. Probenträger zur Untersuchung chemischer und biologischer Proben, der als Kammer (8) mit wenigstens einem Kanal (10) ausgebildet ist, wobei die innere Oberfläche (2) des unteren Teils (12) der Kammer einen transparenten Kunststoff oder Kunststoffverbund umfasst, auf dem an wenigstens einem Bereich (4) der Oberfläche freie Bindungsstellen durch eine Funktionalisierung gebildet werden, die aus wenigstens einer funktionellen Gruppe besteht, **dadurch gekennzeichnet, dass** der untere Teil (12) der Kammer durch eine Folie gegeben ist.

2. Probenträger nach Anspruch 1, wobei die funktionelle Gruppe durch eine Carboxylsäuregruppe (-COOH), eine Aminogruppe (-NH₂), eine Thiolgruppe (-SH), eine Hydroxygruppe (-OH), eine Aldehydgruppe (-CH₂O), eine Säurehalogenidgruppe (-COX) oder einer Polyethylenglycolgruppe gebildet wird.

3. Probenträger nach Anspruch 1 oder 2, wobei der Kunststoff aus der Gruppe der cyclischen Olefinpolymere, der Norbonenpolymere und/oder der cyclischen Olefincopolymere ausgewählt ist und/oder Polypropylen ist.

4. Probenträger nach einem der vorhergehenden Ansprüche, wobei die funktionelle Gruppe an die Polymere chemisch gebunden ist und/oder die Proben durch kovalente Bindung an die funktionellen Gruppen koppelbar sind.

5. Probenträger nach einem der vorhergehenden Ansprüche, wobei an die funktionelle Gruppe der Oberfläche eine oder mehrere weitere reaktive Gruppen, insbesondere aus der Gruppe der Iminodiessigsäure-, Nitrilotriessigsäurederivate oder Biotinderivate oder ein Metall, gebunden sind.

6. Probenträger nach einem der vorhergehenden Ansprüche , wobei der Kunststoff verschiedene Bereiche (4, 4', 4", 4"') mit unterschiedlicher Funktionalisierung aufweist.

7. Probenträger nach einem der vorhergehenden Ansprüche, wobei der Kunststoff keine doppelbrechenden Eigenschaften aufweist und/oder für ultraviolettes Licht durchlässig ist und/oder keine Autofluoreszenzeigenschaften aufweist.

8. Probenträger nach einem der vorhergehenden Ansprüche, wobei er einheitlich aus einem Kunststoff hergestellt ist.

9. Probenträger nach einem der vorhergehenden Ansprüche, wobei der Kunststoff auf ein Probenträgermaterial, insbesondere Glas, aufgebracht ist.

10. Probenträger nach einem der vorhergehenden Ansprüche, wobei alle Oberflächen eines Kanals die Merkmale eines der vorhergehenden Ansprüche aufweisen.

11. Probenträger nach einem der vorhergehenden Ansprüche, wobei in der Kammer verschiedene Kanäle (10, 10', 10", 10"') vorgesehen sind, die jeweils wenigstens eine Kunststoffoberfläche mit einer zu den anderen Kanälen unterschiedlichen Funktionalisierung aufweisen.

12. Probenträger nach einem der vorhergehenden Ansprüche, wobei wenigstens eine Oberfläche eines Kanals verschiedene Abschnitte (6) mit jeweils einer unterschiedlichen Funktionalisierung aufweist.

13. Probenträger nach Anspruch 12, wobei wenigstens einer der Abschnitte (6) eine Funktionalisierung durch ein Metall, insbesondere Gold, aufweist.

14. Probenträger nach einem der vorhergehenden Ansprüche, wobei ein Kanal jeweils eine Zugriffsöffnung als Zulauf (18) und eine als Ablauf (18') für Flüssigkeiten und/oder Gase aufweist und/oder einen Durchmesser von bis zu 2,0 cm aufweist.

15. Probenträger nach einem der vorhergehenden Ansprüche, wobei mehrere Kanäle eine gemeinsame Zugriffsöffnung als Zulauf und eine als Ablauf aufweisen.

## Claims

1. Sample support for assaying chemical and biological samples comprising a chamber (8) with at least one passage (10), wherein the internal surface (2) of the bottom part (12) of the chamber comprises a transparent plastic or plastic composite on which free binding sites are formed at least in a region (4) of the surface by a functionalisation comprising at least one functional group, **characterised in that** the bottom part (12) of the chamber is provided in the form of a film.

2. Sample support as claimed in claim 1, in which the functional group is provided in the form of a carboxylic acid group (-COOH), an amino group (-NH₂), a thiol group (-SH), a hydroxyl group (-OH), an aldehyde group (-CH₂O) an acid halogenide group (-COX) or a polyethylene glycol group.

3. Sample support as claimed in claim 1 or 2, in which the plastic is selected from the group comprising cyclic olefin polymers, norbonene polymers and/or cyclic olefin copolymers and/or is polypropylene.

4. Sample support as claimed in one of the preceding claims, in which the functional group is chemically bonded to the polymers and/or the samples can be coupled by covalent bonding on the functional groups.

5. Sample support as claimed in one of the preceding claims, in which one or more other reactive groups are bonded to the functional group of the surface, in particular from the group comprising imino-diacetic acid derivatives or nitrilo-triacetic acid derivatives or biotin derivatives or a metal.

6. Sample support as claimed in one of the preceding claims, in which the plastic has different regions (4, 4', 4", 4"' ) with differing functionalisation.

7. Sample support as claimed in one of the preceding claims, in which the plastic does not have double-refracting properties and/or is transparent to ultraviolet light and/or has no auto-fluorescence properties.

8. Sample support as claimed in one of the preceding claims, in which it is made uniformly from one plastic.

9. Sample support as claimed in one of the preceding claims, in which the plastic is applied to a sample support material, in particular glass.

10. Sample support as claimed in one of the preceding claims, in which all the surfaces of a passage have the features of one of the preceding claims.

11. Sample support as claimed in one of the preceding claims, in which different passages (10, 10', 10" , 10"') are provided in the chamber, each of which has at least a plastic surface with a functionalisation that is different from that of the other passages.

12. Sample support as claimed in one of the preceding claims, in which at least one surface of a passage has different portions (6), each with a different functionalisation.

13. Sample support as claimed in claim 12, in which at least one of the portions (6) has a functionalisation based on a metal, in particular gold.

14. Sample support as claimed in one of the preceding claims, in which a passage has a respective access opening as an inlet (18) and as an outlet (18') for liquids and/or gases and/or a diameter of up to 2.0 cm.

15. Sample support as claimed in one of the preceding claims, in which several passages have a common access opening as an inlet and as an outlet.

## Revendications

1. Porte-échantillon pour l'analyse d'échantillons chimiques et biologiques, qui est constitué sous la forme d'une chambre (8) comportant au moins un canal (10), la surface interne (2) de la partie inférieure (12) de la chambre comprenant une matière plastique ou matière plastique composite transparente, sur laquelle au niveau d'au moins une région (4) de la surface, des sites de liaison libres sont formés par une fonctionnalisation qui est constituée d'au moins un groupe fonctionnel, **caractérisé en ce que** la partie inférieure (12) de la chambre est constituée par une feuille.

2. Porte-échantillon selon la revendication 1, dans lequel le groupe fonctionnel est formé par un groupe d'acide carboxylique (-COOH), un groupe amino (-NH₂), un groupe thiol (-SH), un groupe hydroxy (-OH), un groupe aldéhyde (-CH₂O), un groupe d'halogénure d'acide (-COX) ou un groupe polyéthylène-glycol.

3. Porte-échantillon selon la revendication 1 ou 2, dans lequel la matière plastique est choisie dans le groupe des polymères d'oléfine cyclique, des polymères de norbornène et/ou des copolymères d'oléfine cyclique et/ou est le polypropylène.

4. Porte-échantillon selon l'une des revendications précédentes, dans lequel le groupe fonctionnel est lié chimiquement au polymère et/ou les échantillons peuvent être couplés par une liaison covalente aux groupes fonctionnels.

5. Porte-échantillon selon l'une des revendications précédentes, dans lequel un ou plusieurs autres groupes réactifs, en particulier issus du groupe des dérivés d'acide imino-diacétique, des dérivés d'acide nitrilo-triacétique ou des dérivés de biotine, ou un métal, sont liés au groupe fonctionnel de la surface.

6. Porte-échantillon selon l'une des revendications précédentes, dans lequel la matière plastique présente différentes régions (4, 4', 4 ", 4"') comportant une fonctionnalisation différente.

7. Porte-échantillon selon l'une des revendications précédentes, dans lequel la matière plastique ne présente pas de propriétés de double réfraction et/ou laisse passer la lumière ultraviolette et/ou ne présente pas de propriété d'autofluorescence.

8. Porte-échantillon selon l'une des revendications précédentes, celui-ci étant produit unitairement à partir d'une matière plastique.

9. Porte-échantillon selon l'une des revendications précédentes, la matière plastique étant appliquée sur un matériau du porte-échantillon, en particulier, le verre.

10. Porte-échantillon selon l'une des revendications précédentes, dans lequel toutes les surfaces d'un canal présentent les caractéristiques de l'une des revendications précédentes.

11. Porte-échantillon selon l'une des revendications précédentes, dans lequel différents canaux (10, 10', 10", 10"') sont prévus dans la chambre, lesquels canaux présentent chacun au moins une surface de matière plastique comportant une fonctionnalisation différente de celle des autres canaux.

12. Porte-échantillon selon l'une des revendications précédentes, dans lequel au moins une surface d'un canal présente différentes sections (6) comportant chacune une fonctionnalisation différente.

13. Porte-échantillon selon la revendication 12, dans lequel au moins l'une des sections (6) présente une fonctionnalisation par un métal, en particulier, l'or.

14. Porte-échantillon selon l'une des revendications précédentes, dans lequel un canal présente respectivement une ouverture d'accès en tant qu'ouverture d'amenée (18) et une ouverture d'accès en tant qu'ouverture d'évacuation (18') pour les liquides et/ou les gaz et/ou présente un diamètre allant jusqu'à 2,0 cm.

15. Porte-échantillon selon l'une des revendications précédentes, dans lequel plusieurs canaux présentent une ouverture d'accès commune en tant qu'ouverture d'amenée et d'évacuation.
